# EUROPÄISCHE PATENTSCHRIFT

(11) **EP 0 907 386 B1**
(45) Veröffentlichungstag und Bekanntmachung des Hinweises auf die Patenterteilung: **05.03.2003**
(21) Anmeldenummer: 97925044.6
(22) Anmeldetag: 04.06.1997
(51) Int. Cl.: A61M 1/10

(54) **BLUTPUMPE**
BLOOD PUMP
POMPE A SANG

(30) Priorität: 25.06.1996 DE 19625300
(43) Veröffentlichungstag der Anmeldung: 14.04.1999
(73) Patentinhaber: MEDOS Medizintechnik AG, 52222 Stolberg (DE)
(72) Erfinder: Rau, Günter, Prof.Dr., 52066 Aachen (DE); Reul, Helmut, Prof. Dr.-Ing., 52353 Düren (DE); Siess, Thorsten, Dr.-Ing., 52146 Würselen (DE)
(74) Vertreter: Selting, Günther, Dipl.-Ing.
(86) Internationale Anmeldenummer: EP9702904
(87) Internationale Veröffentlichungsnummer: WO97049439

(56) Entgegenhaltungen:
- EP-A- 0 611 580
- WO-A-94/09274
- DE-A- 2 113 986
- US-A- 5 503 615
- YOSHINORI MITAMURA ET AL: "THE VALVO-PUMP AN AXIAL, NONPULSATILE BLOOD PUMP" ASAIO TRANSACTIONS, Bd. 37, Nr. 3, 1.Juli 1991, Seiten M510-M512, XP000298549

## Beschreibung

Die Erfindung betrifft eine Blutpumpe, vorzugsweise eine implantierbare Blutpumpe zum Unterstützen oder Ersetzen der links und/oder rechtsseitigen Herzfunktion.

Aus US 4,994,078 ist eine implantierbare Blutpumpe bekannt, die nach Art eines Schneckengehäuses ausgebildet ist, wobei der Stator des Motors in das Pumpengehäuse integriert ist, während der Rotor mit einem Pumpenrad fest verbunden ist und vom Blutstrom umspült wird. Das Blut strömt also durch das Innere des Motors hindurch, wobei die eine Wand des von dem Motor gebildeten Ringkanals stationär ist, während die andere Wand rotiert. Dadurch besteht dort die Gefahr von Blutschädigungen durch auftretende Scherkräfte.

EP 0 611 580 A2 beschreibt eine künstliche Herzpumpe, bei der ebenfalls der Rotor und der Stator des Motors vom Blut umspült sind. An der Auslaßseite befindet sich ein Pumpenrad, das als Radialpumpe ausgebildet ist und axial entlang des Stators angesaugtes Blut in einen umgebenden Ringkanal hineinpumpt, aus dem das Blut tangential austritt. Hierbei bestehen zwei Ansaugwege, nämlich der den Stator umgebende Hauptansaugweg und ein relativ enger Ringkanal zwischen Stator und Rotor. Auch hierbei besteht die Gefahr von Blutschädigung durch Scherkräfte.

Schließlich ist aus WO 94/09274 eine Blutpumpe bekannt, die einen schraubenförmigen Rotor aufweist, welcher einen Stator umgibt.

Der Oberbegriff des Patentanspruchs 1 geht aus von einer Blutpumpe, wie sie aus DE-A-2113986 bekannt ist. Bei dieser bekannten Blutpumpe strömt das Blut zwischen statischen Wänden hindurch, die von einem Pumpengehäuse und einem darin angeordneten Statorkörper gebildet werden. Das Pumpengehäuse ist ei-förmig. Dieser Eiform entsprechend ist das Statorgehäuse ebenfalls ei-förmig. Das Statorgehäuse bildet eine Kapselung für einen Elektromotor. Es ist in Anpassung an das Pumpengehäuse geformt. Dies hat zur Folge, dass der Elektromotor innerhalb des Statorgehäuses so angeordnet ist, dass er nicht in direkten wärmeleitenden Kontakt mit dem strömenden Blut kommt. Zwischen dem Statorgehäuse und dem Pumpengehäuse strecken sich Statorschaufeln, die den Stator im Pumpengehäuse halten. Diese Schaufeln bilden längslaufende Einbauten.

Der Erfindung liegt die Aufgabe zugrunde, eine Blutpumpe zu schaffen, in der Blutschädigungen und Thrombenbildung weitgehend vermieden werden.

Die Lösung dieser Aufgabe erfolgt erfindungsgemäß mit den im Patentanspruch 1 angegebenen Merkmalen.

Bei der erfindungsgemäßen Blutpumpe ist in einem langgestreckten Pumpengehäuse der Motor als geschlossene Einheit koaxial untergebracht, wobei zwischen dem Pumpengehäuse und dem Motorgehäuse ein Ringkanal gebildet wird, der von dem Blut durchströmt wird. Das Pumpenrad ist am einlaßseitigen Ende des Motors angeordnet und bildet zusammen mit einem Übergangsteil des Pumpengehäuses eine diagonal durchströmte Pumpe, die einen das Motorgehäuse ringförmig umspülenden, drallbehafteten Axialstrom erzeugt. Die Blutströmung erfolgt also im Bereich des Motors zwischen zwei stationären Teilen, nämlich dem Pumpengehäuse und dem Motorgehäuse, wodurch zusätzliche Scherbeanspruchungen des Blutes außerhalb des rotierenden Teils der Pumpenbeschaufelung vermieden werden. Der an dem Motorgehäuse entlangströmende Blutstrom bewirkt eine ständige Kühlung des Motors, solange die Pumpe arbeitet. Der Motor wird also durch das an ihm entlangströmende Blut gekühlt. Das Pumpengehäuse ist langgestreckt und im wesentlichen rohrförmig und ohne rotierend umlaufende Ringkanäle oder sonstige Verdickungen. Dies ermöglicht eine einfache und raumsparende Implantation in Herznähe unter Berücksichtigung der physiologischen Gegebenheiten. Der axiale Blutstrom entlang des Pumpengehäuses weist eine Umfangskomponente auf, wobei das Blut das Motorgehäuse schraubenförmig umströmt.

Das Pumpengehäuse hat vorzugsweise eine Länge von etwa 5 bis 10 cm und einen Außendurchmesser von etwas 2 bis 3 cm. Der Einlaß und der Auslaß sind, bezogen auf den Außendurchmesser des Pumpengehäuses, verjüngt ausgebildet.

Bei einer bevorzugten Ausführungsform der Blutpumpe führt der Auslaß seitlich aus dem Pumpengehäuse heraus. Hierbei kann am auslaßseitigen Ende des Pumpengehäuses eine Stirnwand vorgesehen sein, die einen zu dem Auslaß führenden schraubenförmigen Kanal mit dem Winkel der dortigen Strömung enthält. Eine solche Blutpumpe eignet sich insbesondere für Patienten mit irreversiblem linksventrikulären Herzversagen. Dabei wird eine Anschlußstelle in den Ventrikel gelegt, so daß dieser durch Blutentnahme entlastet wird. Mit der Anschlußstelle wird der Pumpeneinlaß verbunden, während der Pumpenauslaß an die Aorta angeschlossen wird. Der um ca. 90° gegenüber dem Einlaß gewinkelte Auslaß dieser Ausführungsform ist physiologisch auch für eine Vorhof-Aorta-Kanüllierung günstig.

Der Anschluß des Blutpumpeneinlasses an den Vorhof kann nach der Entfernung des Anschlusses wieder verschlossen werden. Das Herz kann also seine eigenständige Arbeit wieder aufnehmen. Diese Methode wird dann bevorzugt, wenn die Möglichkeit einer langfristigen Herzerholung besteht oder die Pumpe als chronische Unterstützungspumpe des vermindert, aber noch aktiven Herzens dienen soll.

Bei einer anderen Ausführungsform der Blutpumpe führt der Auslaß axial aus dem Pumpengehäuse heraus.

Im folgenden werden unter Bezugnahme auf die Zeichnungen Ausführungsbeispiele der Erfindung näher erläutert.

Es zeigen:
- Fig. 1: einen Längsschnitt durch eine erste Ausführungsform der Blutpumpe,
- Fign. 2a und 2b: schematische Darstellungen des Einsatzes der Blutpumpe am Herzen, und
- Fig. 3: eine zweite Ausführungsform der Blutpumpe mit axial gerichtetem Einiaß und Auslaß.

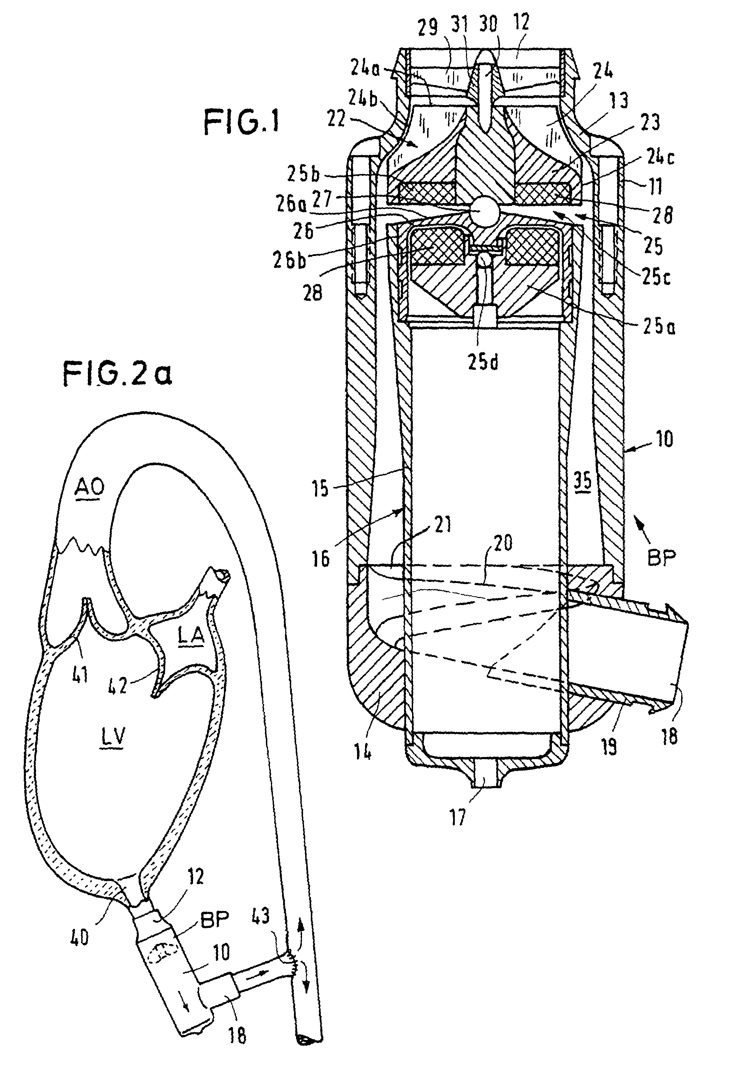

Die Blutpumpe BP nach Fig. 1 weist ein langgestrecktes, rohrförmiges Pumpengehäuse 10 auf, das an einem Ende mit einem Einlaßteil 11 versehen ist. Der Einlaßteil 11 weist einen Einlaß 12 auf, dessen Innendurchmesser kleiner ist als der Innendurchmesser des Pumpengehäuses 10. Im Einlaßteil 11 ist ein ringförmiger Übergangsteil 13 ausgebildet, der bogenförmig gestaltet ist und in einer S-förmigen Krümmung von dem Durchmesser des Einlasses 12 auf den Innendurchmesser des Pumpengehäuses 10 glatt und knickfrei überleitet.

An dem dem Einlaß 12 gegenüberliegenden Ende des Pumpengehäuses 10 befindet sich eine Stirnwand 14, die das Pumpengehäuse abschließt. An dieser Stirnwand 14 ist das Motorgehäuse 15 eines Motors 16 befestigt, bei dem es sich um einen Elektromotor handelt. Das Motorgehäuse 15 ragt aus der Stirnwand 14 heraus. Es ist vollständig abgedichtet, wobei die elektrischen Leitungen durch einen Durchlaß 17 des Motorgehäuses 15 hindurchgeführt sind.

Der Auslaß 18 befindet sich an einem Auslaßstutzen 19, der tangential von der Stirnwand 14 seitlich abgeht. Im Innern der Stirnwand 14 verläuft ein schraubenförmiger Kanal 20 in Richtung der drallbeaufschlagten Axialströmung von 21 bis zum Auslaß 18 mit ausschließlich axialer Erstreckung.

In dem Einlaßteil 11 des Pumpengehäuses 10 ist ein Pumpenrad 22 angeordnet. Dieses besteht aus einer Nabe 23, von der Schaufeln 24 abstehen. Der Antrieb des Pumpenrades 22 erfolgt von dem Motor 16 über eine magnetische Kupplung, die als Stirndrehkupplung 25 oder Zentraldrehkupplung ausgebildet sein kann. Diese weist ein erstes Kupplungsteil 25a auf, das mit dem Rotor des Motors 16 verbunden und im Innern des Motorgehäuses 15 gekapselt angeordnet ist, und ein zweites Kupplungteil 25b, das in der Nabe 23 des Pumpenrades 22 angeordnet ist. Beide Kupplungsteile 25a,25b haben Magnete 28, die bewirken, daß bei einer Drehung des erstes Kupplungsteils 25a das zweite Kupplungsteil 25b mitdreht.

Das Motorgehäuse 15 ist mit einer amagnetischen nichtleitenden Kappe 26 verschlossen, an welcher ein kombiniertes Axial/Radiallager 27 in Form einer Kugel abgestützt ist. Dieses Lager 27 stützt seinerseits die Nabe des Pumpenrades 22 ab. Die Magnete 28 der Stirndrehkupplung 25 erzeugen eine axiale Haltekraft, die größer ist als die beim Drehen des Pumpenrades 22 erzeugte Reaktionskraft, so daß das Pumpenrad 22 durch die Magnetkraft in Richtung auf den Motor 16 gezogen und gegen das Lager 27 gedrückt wird. Die axiale Haltekraft der Kupplung 25 wird zentral auf der Kappe 26 durch eine weitere axiale Lagerabstützung 25d in Verbindung mit dem Lager 27 kompensiert, so daß weder die Lager des Motors 26 noch die dünne Kappe 26 diese Kraft in ihrer Umfangswand 26b und auf der Stirnfläche 26a aufnehmen müssen.

Zur Zentrierung des Pumpenrades 22 auf der Einlaßseite ist in dem Einlaß 12 ein Armstern 29 befestigt, der einen axial in die Nabe 23 des Pumpenrades 22 eingreifenden Lagerzapfen 30 aufweist. An dem Armstern 29 befindet sich ein zentrisches Kopfstück 31, das den Lagerzapfen 30 umgibt und die axial einströmende Strömung geringfügig radial in Richtung der Nabe 23 ableitet.

Die Leitschaufeln 24 des Pumpenrades 22 haben an dem Anströmende 24a einen Außendurchmesser, der im wesentlichen dem Durchmesser des Einlasses 12 entspricht, so daß das Pumpenrad hier den gesamten Durchmesser des Einlaßkanals erfaßt. Im Anschluß an die Einlaßkanten 24a erfolgt ein konkav-bogenförmiger Umfangsbereich 24b, der dem Übergangsteil 13 des Pumpengehäuses mit geringem Abstand folgt. Daran anschließend haben die Pumpenschaufeln 24 einen Bereich 24c, dessen Durchmesser etwa so groß ist wie der Außendurchmesser des Motorgehäuses 15 an demjenigen Ende, das dem Pumpenrad 22 zugewandt ist. Der Außendurchmesser der diagonalen Pumpenschaufeln 24 wird so groß ausgeführt, wie es der Gesamtdurchmesser des Übergangsteils 13 erlaubt. Hierdurch kann die erforderliche, hydraulische Leistung bei einer vergleichsweisen geringen Drehzahl (beispielhaft n≅7000U/min für V/t≅5l/min und ΔP≅100mmHg) erzielt werden, was die Standzeiten der lagernden Komponenten der Pumpe erhöht.

Zwischen dem Pumpengehäuse 10 und dem Motorgehäuse 15 besteht ein Ringkanal 35, der sich in Längsrichtung erstreckt. Dieser Ringkanal 35 ist als Diffusor ausgebildet, indem sich seine Querschnittsfläche vom Einlaß zum Auslaß vergrößert. Dadurch entsteht eine Verlangsamung des Blutflusses und somit eine Druckerhöhung. Die Erweiterung der Querschnittsfläche des Ringkanals 35 wird durch entsprechende Veränderung der Wandstärken von Pumpengehäuse 10 und Motorgehäuse 15 erreicht.

Der Motor 16 bewirkt eine Rotation des Pumpenrades 22, dessen Schaufelräder 24 schraubenförmig gestaltet sind. Dadurch wird Blut aus dem Einlaß 12 axial angesaugt und mit einer rotierenden Umfangskomponente in den Ringkanal 25 gefördert. Von dort gelangt das Blut durch den schraubenförmigen Kanal 20, dessen Verlauf und Steigung demjenigen des rotierenden Blutstroms angepaßt ist zum seitlichen Auslaß 18.

Fig. 2a zeigt die Blutpumpe BP nach Fig. 1 in dem Zustand, in dem sie am Herzen eingesetzt wird. Der Einlaß 12 der Blutpumpe BP wird über einen Schlauch mit einem an der linken Herzkammer LV erzeugten Zugang 40 verbunden. In der Zeichnung sind die Aorta AO, die Aortenklappe 41, der linke Vorhof LA und die Mitralklappe 42 erkennbar. Der Auslaß 18 der Blutpumpe BP wird mit einem an der Aorta AO geschaffenen Zugang 43 verbunden.

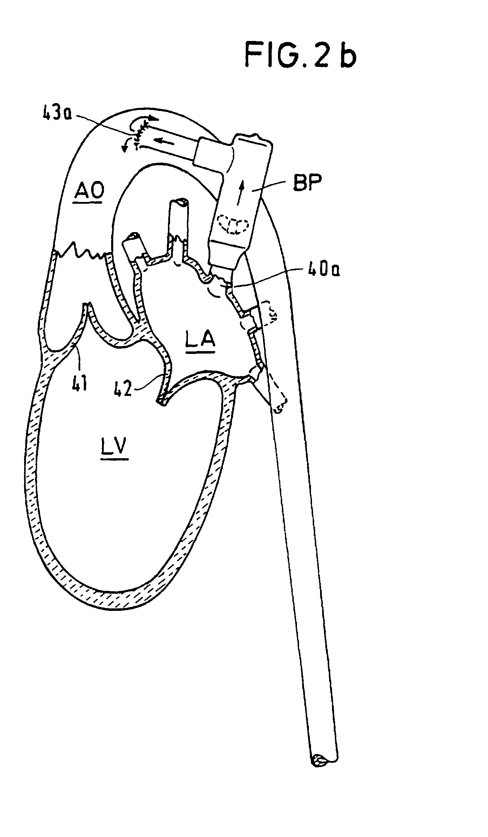

Alternativ kann die Blutpumpe BP gemäß Fig. 2b über einen am Vorhof LA erzeugten Zugang 40a verbunden werden und Blut über einen Zugang 43a in die Aorta AO fördern.

Aus Fig. 2 ist erkennbar, daß der seitliche Abgang des Auslasses 18 von dem Pumpengehäuse 10 die Anordnung der Blutpumpe am Herzen und ihren Anschluß an das Herz/Vorhof einerseits und die Aorta andererseits erleichtert.

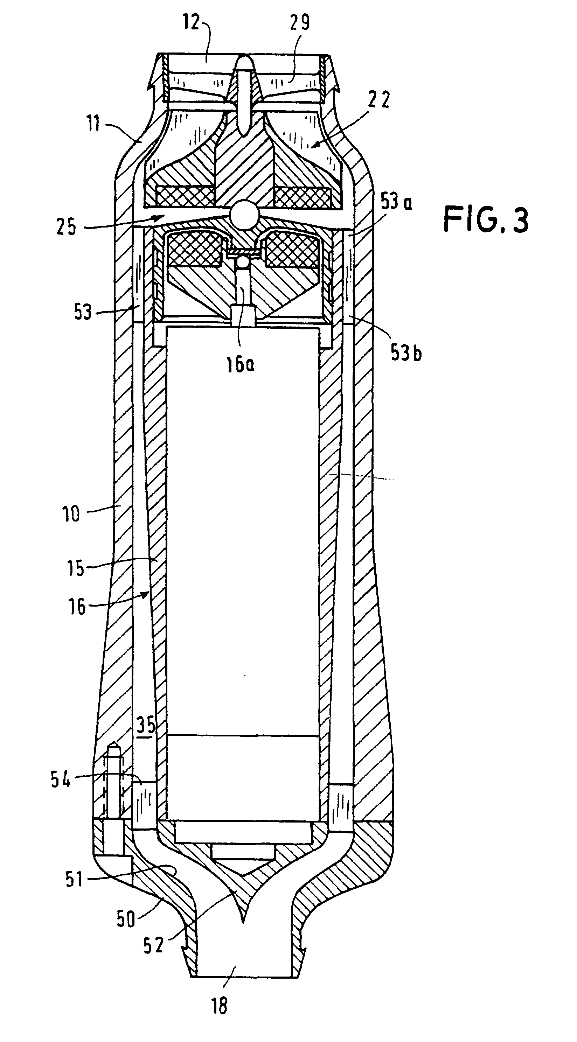

Bei der in Fig. 3 dargestellten Blutpumpe ist der Auslaß 18 koaxial zum Einlaß 12 angeordnet. Er ist Bestandteil eines Auslaßteiles 50, der ähnlich wie der Einlaßteil 11 ausgebildet ist und einen bogenförmigen Übergangsbereich 51 aufweist, der von dem Ringkanal 35 zu dem Auslaß 18 in strömungsgünstiger Weise überleitet. Zu dem gleichen Zweck ist an dem rückwärtigen Ende des Motorgehäuses 15 ein sich in Strömungsrichtung stetig verjüngender Ansatz 52 vorgesehen.

Um das Motorgehäuse 15 zentrisch in dem Pumpengehäuse 10 zu halten, sind radiale Leitschaufeln 53,54 vorgesehen, deren Funktion einerseits darin besteht, das Motorgehäuse zu halten, und andererseits darin, die rotierende Blutströmung zu leiten. Die Leitschaufeln 53 haben (in Bezug auf die Axialrichtung) einen relativ großen Anstellwinkel im Bereich 53a, der so gewählt ist, daß die Leitschaufeln 53 ohne Strömungsablösung angeströmt werden. Im hinteren Bereich 53b ist der Anstellwinkel kleiner. Hierdurch wird dem aus dem Pumpenrad 22 kommenden Blut ein Großteil der Rotationsenergie entzogen und gezielt in Druck umgewandelt. Die Leitschaufeln 54 haben einen geringeren Anstellwinkel. Die Anstellwinkel entsprechen im wesentlichen der Strömungsrotation an den jeweiligen Stellen.

Im übrigen ist die Blutpumpe von Fig. 3 in gleicher Weise ausgebildet wie die Blutpumpe nach Fig. 1. Sie ist mit einer Magnetkupplung 25 für den Antrieb des Pumpenrades 22 versehen. Alternativ besteht die Möglichkeit, das Pumpenrad 22 direkt mit der Motorwelle 16a zu verbinden, jedoch ist dann eine Wellendichtung am Motorgehäuse erforderlich.

## Patentansprüche

1. Blutpumpe zum Unterstützen oder Ersetzen der Herzfunktion, mit einem Pumpengehäuse (10), in dem ein Motor (16), der ein Pumpenrad (22) antreibt, angeordnet ist und das an einem Ende einen Einlaß (12) und am entgegengesetzten Ende einen Auslaß (18) aufweist,
wobei der Motor (16) ein in dem Pumpengehäuse (10) enthaltenes, gegen den Blutstrom abgedichtetes, konzentrisch angeordnetes Motorgehäuse (15) aufweist und das Pumpenrad (22) an dem dem Einlaß (12) zugewandten Ende des Motorgehäuses (15) angeordnet ist und mit einem Übergangsteil (13) des Pumpengehauses (10) eine Rotationspumpe bildet, die einen das Motorgehäuse (15) ringförmig umspülenden Axialstrom erzeugt, und wobei der Axialstrom von stationären Wänden umspannt wird,
**dadurch gekennzeichnet,**
**daß** das Pumpengehäuse (10) langgestreckt und rohrförmig ist und daß zwischen Pumpengehäuse (10) und Motorgehäuse (15) ein eine drallbehaftete schraubenförmige Strömung leitender Ringkanal (35) vorhanden ist.

2. Blutpumpe nach Anspruch 1, **dadurch gekennzeichnet, daß** der Auslaß (18) axial aus dem Pumpengehäuse (10) herausführt.

3. Blutpumpe nach Anspruch 1, **dadurch gekennzeichnet, daß** der Auslaß (18) tangential seitlich aus dem Pumpengehäuse (10) herausführt.

4. Blutpumpe nach Anspruch 3, **dadurch gekennzeichnet, daß** am auslaßseitigen Ende des Pumpengehäuses (10) eine Stirnwand (14) vorgesehen ist, die einen zu dem Auslaß (18) führenden schraubenförmigen Kanal (20) enthält.

5. Blutpumpe nach einem der Ansprüche 1-4, **dadurch gekennzeichnet, daß** das Motorgehäuse (15) frei abstehend an einer Stirnwand (14) befestigt ist.

6. Blutpumpe nach Anspruch 4, **dadurch gekennzeichnet, daß** der zum Auslaß führende schraubenförmige Kanal (20) in den Auslaß (18) überleitet, ohne den Durchmesser des Pumpengehäuses (10) zu vergrößern.

7. Blutpumpe nach Anspruch 4, **dadurch gekennzeichnet, daß** die axiale Steigung des schraubenförmigen Kanals (20) derjenigen der drallbehafteten Strömung entspricht, so daß das Fluid stoßfrei in den Auslaß (18) übergeleitet wird.

8. Blutpumpe nach einem der Ansprüche 1 bis 7, **dadurch gekennzeichnet, daß** der Ringkanal (35) als Diffusor ausgebildet ist, dessen Querschittsfläche sich in Richtung auf den Auslaß (18) vergrößert.

9. Blutpumpe nach einem der Ansprüche 1 bis 8, **dadurch gekennzeichnet, daß** in dem Ringkanal (35) zwischen Pumpengehäuse (10) und Motorgehäuse (15) Leitschaufeln (53,54) angeordnet sind, die das Motorgehäuse (15) halten und eine schraubenförmige Schrägstellung aufweisen.

10. Blutpumpe nach einem der Ansprüche 1 bis 9, **dadurch gekennzeichnet, daß** der größte Durchmesser des Pumpenrades (22) wenigstens annähernd gleich dem größten Außendurchmesser des Motorgehäuses (15) ist.

11. Blutpumpe nach einem der Ansprüche 1 bis 10, **dadurch gekennzeichnet, daß** das Pumpengehäuse (10) über seine gesamte Länge eine im wesentlichen zylindrische Außenform hat, wobei ggf. der Einlaß (12) und/oder der Auslaß (18) verjüngt ausgebildet ist.

12. Blutpumpe nach einem der Ansprüche 1 bis 11, **dadurch gekennzeichnet, daß** das Moment des Motors (16) über eine Magnetkupplung (25) auf das Pumpenrad (22) übertragen wird.

13. Blutpumpe nach einem der Ansprüche 1 bis 11, **dadurch gekennzeichnet, daß** das Moment direkt vom Motor (16) über eine durchgehende Welle mit Dichtung auf das Pumpenrad (22) übertragen wird.

14. Blutpumpe nach Anspruch 13, **dadurch gekennzeichnet, daß** das Pumpenrad in Blut mechanisch (27,63) zentriert wird.

15. Blutpumpe nach Anspruch 12, **dadurch gekennzeichnet, daß** das Pumpenrad (22) in Blut durch eine Kombination aus magnetischer und mechanischer Lagerung (27) zentriert wird.

16. Blutpumpe nach Anspruch 12, **dadurch gekennzeichnet, daß** die axiale Kraft der Kupplung direkt zentral auf der Kappe (26) kompensiert wird, wodurch dieses Bauteil und die Lager des Motors (16) axial deutlich geringer belastet werden.

## Claims

1. A blood pump for supporting or replacing the cardiac function, comprising a pump housing (10) in which a motor (16) is arranged that drives a pump impeller wheel (22), and which has an inlet (12) at one end and an outlet (18) at the other end,
the motor (16) comprising a motor housing (15) concentrically arranged in the pump housing (10) and sealed off against the blood flow, the pump impeller wheel (22) being arranged at the end of the motor housing (15) that faces the inlet (12) and forming a rotary pump with a transition portion (13) of the pump housing (10), which pump creates an axial flow annularly flowing about the motor housing (15), and said axial flow being enclosed by stationary walls,
**characterized in**
**that** the pump housing (10) is elongate and tubular and that an annular channel (35) directing a whirling helical flow is provided between the pump housing (10) and the motor housing (15).

2. The blood pump of claim 1, **characterized in that** the outlet (18) exits axially from the pump housing (10).

3. The blood pump of claim 1, **characterized in that** the outlet (18) exits tangentially from the side of the pump housing (10).

4. The blood pump of claim 3, **characterized in that** an end wall (14) is provided at the outlet end of the pump housing (10), the wall comprising a helical channel (20) leading to the outlet (18).

5. The blood pump of one of claims 1-4, **characterized in that** the motor housing (15) is fastened to the end wall (14) in a cantilevered manner.

6. The blood pump of claim 4, **characterized in that** the helical channel (20) leading to the outlet (18) passes into the outlet (18) without increasing the diameter of the pump housing (10).

7. The blood pump of one of claims 4, **characterized in that** the axial pitch of the helical channel (20) corresponds to that of the whirling flow so that the fluid is passed into the outlet (18) without any impact.

8. The blood pump of one of claims 1 to 7, **characterized in that** the annular channel (35) is formed as a diffusor, the sectional area of which increases towards the outlet (18).

9. The blood pump of one of claims 1 to 8, **characterized in that** guide blades (53, 54) are arranged in the annular channel (35) between the pump housing (10) and the motor housing (15), the blades holding the motor housing (10) and having a helical inclination.

10. The blood pump of one of claims 1 to 9, **characterized in that** the largest diameter of the pump impeller wheel (22) is at least almost equal the largest outer diameter of the motor housing (15).

11. The blood pump of one of claims 1 to 10, **characterized in that** the pump housing (10) has a substantially cylindrical outer shape over its entire length, the inlet (12) and/or the outlet (18) possibly being tapered.

12. The blood pump of one o claims 1 to 11, **characterized in that** the moment of the motor (16) is transmitted to the pump impeller wheel (22) via a magnetic clutch (25).

13. The blood pump of one o claims 1 to 11, **characterized in that** te moment is transferred directly from the motor (16) to the pump impeller wheel (22) via a continuous shaft with a seal.

14. The blood pump of claim 13, **characterized in that** the pump impeller wheel is mechanically centered (27, 63) in the blood.

15. The blood pump of claim 12, **characterized in that** the pump impeller wheel (22) is centered **in that** blood by a combination of magnetic and mechanic bearing (27).

16. The blood pump of claim 12, **characterized in that** the axial force of the clutch is compensated directly on the center of a cap (26), whereby this component and the bearings of the motor (16) are subjected to substantially less axial loading.

## Revendications

1. Pompe à sang pour soutenir ou remplacer la fonction du coeur, comprenant un carter de pompe (10) dans lequel est disposé un moteur (16) entraînant une roue de pompe (22) et qui présente une entrée (12) à une extrémité et une sortie (18) à l'extrémité opposée, le moteur (16) présentant un carter de moteur (15) étanche au flux de sang contenu et disposé concentriquement dans le carter de pompe (10), et la roue de pompe (22) étant disposée à l'extrémité du carter de moteur (15) tournée vers l'entrée (12) et constituant conjointement avec un élément de transition (13) du carter de pompe (10) une pompe rotative, qui engendre un flux axial entourant de façon annulaire le carter de moteur (15), le flux axial étant enserré dans des parois stationnaires, **caractérisée en ce que** le carter de pompe (10) est allongé et tubulaire, et **en ce qu'**entre le carter de pompe (10) et le carter de moteur (15) est disposé un canal annulaire (35) conduisant un flux giratoire en forme de vis.

2. Pompe à sang selon la revendication 1, **caractérisée en ce que** la sortie (18) saillit axialement hors du carter de pompe (10).

3. Pompe à sang selon la revendication 1, **caractérisée en ce que** la sortie (18) saillit hors du carter de pompe latéralement de façon tangentielle.

4. Pompe à sang selon la revendication 3, **caractérisée en ce qu'**à l'extrémité côté sortie du carter de pompe (10) est prévue une paroi frontale (14) qui contient un canal (20) en forme de vis menant vers la sortie (18).

5. Pompe à sang selon l'une des revendications 1-4, **caractérisée en ce que** le carter de moteur (15) est fixé en saillie libre sur une paroi frontale (14).

6. Pompe à sang selon la revendication 4, **caractérisée en ce que** le canal (20) en forme de vis menant vers la sortie débouche dans la sortie (18) sans agrandir le diamètre du carter de pompe (10).

7. Pompe à sang selon la revendication 4, **caractérisée en ce que** le pas axial du canal (20) en forme de vis correspond à celui du flux giratoire de telle sorte que le fluide est conduit sans à-coups dans la sortie (18).

8. Pompe à sang selon l'une des revendications 1 à 7, **caractérisée en ce que** le canal annulaire (35) est configuré sous la forme d'un diffuseur dont la superficie de section s'agrandit en direction de la sortie (18).

9. Pompe à sang selon l'une des revendications 1 à 8, **caractérisée en ce qu'**à l'intérieur du canal annulaire (35), entre le carter de pompe (10) et le carter de moteur (15), sont disposées des aubes directrices (53, 54) qui maintiennent le carter de moteur (15) et qui présentent une position inclinée en forme de vis.

10. Pompe à sang selon l'une des revendications 1 à 9, **caractérisée en ce que** le plus grand diamètre de la roue de pompe (22) est au moins sensiblement égal au plus grand diamètre extérieur du carter de moteur (15).

11. Pompe à sang selon l'une des revendications 1 à 10, **caractérisée en ce que** le carter de pompe (10) présente sur toute sa longueur une forme extérieure pour l'essentiel cylindrique, l'entrée (12) et/ou la sortie (18) étant le cas échéant configurée à section décroissante.

12. Pompe à sang selon l'une des revendications 1 à 11, **caractérisée en ce que** le couple du moteur (16) est transmis à la roue de pompe (22) par l'intermédiaire d'un accouplement magnétique (25).

13. Pompe à sang selon l'une des revendications 1 à 11, **caractérisée en ce que** le couple est transmis sur la roue de pompe (22) directement par le moteur (16) par l'intermédiaire d'un arbre continu avec joint d'étanchéité.

14. Pompe à sang selon la revendication 13, **caractérisée en ce que** la roue de pompe est mécaniquement (27, 63) centrée dans le sang.

15. Pompe à sang selon la revendication 12, **caractérisée en ce que** la roue de pompe (22) est centrée dans le sang à l'aide d'une combinaison de paliers (27) magnétiques et mécaniques.

16. Pompe à sang selon la revendication 12, **caractérisée en ce que** la force axiale de l'accouplement est compensée directement au centre du capot (26), permettant une sollicitation axiale considérablement réduite de cet élément et des paliers du moteur (16).
